# EUROPEAN PATENT APPLICATION

(11) **EP 4 299 718 A1**
(43) Date of publication of application: **03.01.2024**
(21) Application number: 22460029.6
(22) Date of filing: 30.06.2022
(51) Int. Cl.: C12N 1/20, C12R 1/01, C12R 1/25

(54) **LACTIC ACID BACTERIAL STRAINS OF LATILACTOBACILLUS CURVATUS, LIMOSILACTOBACILLUS FERMENTUM AND LACTIPLANTIBACILLUS PLANTARUM FOR ANTIMICROBIAL APPLICATIONS, METHOD FOR ISOLATING SAID STRAINS AND METHOD FOR OBTAINING SILVER NANOPARTICLES, SILVER CHLORIDE NANOPARTICLES AND HYBRIDS OF SILVER AND SILVER CHLORIDE NANOPARTICLES, AND ZINC OXIDE NANOPARTICLES FROM SAID STRAINS**

(30) Priority: 29.06.2022 PL 44158522
(71) Applicant: Uniwersytet Mikolaja Kopernika W Toruniu, 87-100 Torun (PL)
(72) Inventor: Buszewski, Boguslaw, 87-134 Stary Torun (PL); Railean-Plugaru, Viorica, 87-100 Torun (PL); Król-Górniak, Anna, 87-100 Torun (PL); Pomastowski, Pawel, 87-134 Przysiek (PL)

(57) **Abstract**

The objects of the inventions are new lactic acid bacterial strains *Latilactobacillus curvatus, Limosilactobacillus fermentum* and *Lactiplantibacillus plantarum* for antimicrobial applications, a method for isolating the lactic acid bacterial strains *Latilactobacillus curvatus, Limosilactobacillus fermentum* and *Lactiplantibacillus plantarum* and a method for obtaining silver nanoparticles, silver chloride nanoparticles and hybrids of silver nanoparticles and silver chloride and zinc oxide nanoparticles from the lactic acid bacterial strain *Latilactobacillus curvatus, Limosilactobacillus fermentum* and *Lactiplantibacillus plantarum.*

## Description

New lactic acid bacterial strains of *Latilactobacillus curvatus, Limosilactobacillus fermentum* and *Lactiplantibacillus plantarum* for antimicrobial applications, the method for isolating lactic acid bacterial strains of *Latilactobacillus curvatus, Lactiplantibacillus plantarum* and *Limosilactobacillus fermentum* and the method for obtaining silver nanoparticles, silver chloride nanoparticles and hybrids of silver and silver chloride nanoparticles, and zinc oxide nanoparticles from the lactic acid bacterial strains of *Latilactobacillus curvatus, Lactiplantibacillus plantarum* and *Limosilactobacillus fermentum.*

The subject of the invention is new lactic acid bacterial strains of *Latilactobacillus curvatus, Limosilactobacillus fermentum* and *Lactiplantibacillus plantarum* for antimicrobial applications. The subject of the invention is also the method for isolating lactic acid bacterial strains of *Latilactobacillus curvatus, Limosilactobacillus fermentum* and *Lactiplantibacillus plantarum.* The subject of the invention is also the method for obtaining silver and silver chloride nanoparticles, as well as hybrids of silver and silver chloride nanoparticles and zinc oxide nanoparticles from the lactic acid bacterial strains of *Latilactobacillus curvatus, Limosilactobacillus fermentum* and *Lactiplantibacillus plantarum.*

A method for isolating new strains of lactic acid bacteria and a method for obtaining silver nanoparticles using these strains, as well as the therapeutic use of such silver nanoparticles, is known from the Polish invention application P.418480. The subject of the application is new lactic acid bacterial strains of *Lactobacillus casei* isolated from sea cheese produced in the Drzycim region, *Bifidobacterium sp.* isolated from milk produced in the Drzycim region, *Lactococcus lactis* 56 and 58 isolated from milk produced in the Drzycim region. The application also includes a method for producing silver nanoparticles characterised by the fact that it uses pre-isolated new strains of lactic bacteria and is carried out extracellularly using supernatants obtained after culturing *Lactobacillus casei, Bifidobacterium sp., Lacfococcus lactis* 56 and 58.

A method for producing a composition containing colloidal metal nanoparticles, including silver, gold, zinc, mercury, copper, palladium, platinum or bismuth involving contacting a metal or metal compound with bacteria is known from the US patent US8455226B2. The method also includes a stage of incubating the probiotic bacteria with an aqueous solution containing at least 4 mM silver or gold salts. The resulting composition containing nanosilver is used as a highly effective antimicrobial agent.

A method for obtaining antibacterial zinc oxide nanocomposites by extracellular biosynthesis using a supernatant obtained from the culture of a strain of lactic acid bacteria and a precursor in the form of zinc nitrate is characterised by the fact that the strain of lactic acid bacteria is *Lactobacillus paracasei* strain LPC20 deposited under No. B/00287 and zinc nitrate is added to the post-culture supernatant with simultaneous stirring at a concentration of 0.1 g/mL. The biosynthesis process is carried out at 60 °C for 1 h after which the supernatant is heated at 100 °C until the liquid is fully evaporated and the nanocomposite is obtained in the powder form. The nanocomposite is then purified by rinsing three times with deionised water accompanied by centrifugation.

A method for producing nanometric zinc oxide based on a strain of *Lactobacillus casei* is known from the Chinese invention application CN110104673A. The method serves to solve the technical problem of low purity of nanometric zinc oxide produced by known methods. The method comprises the following stages: preparation of an intermediate of Zn(OH)2 using anhydrous zinc acetate as the substrate and *Lactobacillus casei* L. Casei ATCC 393 as the biological medium, magnetic stirring of an intermediate of Zn(OH)2 at a constant temperature followed by calcination of the stirred intermediate compound at a high temperature to obtain nano-ZnO. The method according to the invention has the following advantages: anhydrous zinc acetate is less toxic than ZnCl2, *Lactobacillus casei,* L. Casei ATCC 393 is safer than *Lactobacillus saliva* L3, and the production of nano-ZnO is based on the fermentation product of L. casei ATCC 393 rather than the bacterium itself so the separation and purification process is simplified, and thus the yield and purity of the prepared nano-ZnO product are high.

A method for biosynthesising zinc nanoxide using lactic acid bacteria and a composite addictive agent in the form of zinc nanoxide is known from the Chinese invention application CN103602707. The invention uses lactic acid bacteria to synthesise zinc nanoxide, and further demonstrates a novel feed addictive agent that is rich in zinc nanoxide. The addictive substance is administered to the feed of suckling piglets at less than 1,000 ppm zinc oxide by weight. The addictive can effectively prevent zinc poisoning of animals and reduce environmental pollution caused by zinc. It can also effectively prevent diarrhoea in weaner pigs and improve gastrointestinal health through the co-function of lactic acid bacteria and zinc oxide, and can even completely or partially replace an antibiotic to reduce antibiotic residues to improve food safety.

The purpose of the invention is to develop such strains of bacteria from cow milk *Latilactobacillus curvatus, Limosilactobacillus fermentum* and *Lactiplantibacillus plantarum* that allow the synthesis of zinc oxide, silver, silver chloride nanoparticles and hybrid systems of silver and silver chloride nanoparticles.

Another purpose of the invention is to develop a method for isolating lactic acid bacterial strains of *Latilactobacillus curvatus, Limosilactobacillus fermentum* and *Lactiplantibacillus plantarum,* which will allow the synthesis of zinc oxide, silver, silver chloride nanoparticles and hybrid systems of silver and silver chloride nanoparticles.

Another purpose of the invention is to provide a method for obtaining silver nanoparticles, silver chloride nanoparticles and hybrids of silver and silver chloride nanoparticles from lactic acid bacterial strains of *Latilactobacillus curvatus, Limosilactobacillus fermentum and Lactiplantibacillus plantarum,* which will allow the synthesis of zinc oxide, silver nanoparticles, silver chloride nanoparticles and hybrid systems of silver and silver chloride nanoparticles.

The essence of the invention concerns a lactic acid bacterial strain of *Latilactobacillus curvatus* deposited in the Polish Collection of Microorganisms (PCM) at the Institute of Immunology and Experimental Therapy of the Polish Academy of Sciences, 12 Rudolf Weigl St. with its seat in Wroclaw, Poland, under number [B/00399] for use in the synthesis of zinc oxide, silver and silver chloride nanoparticles and hybrid systems of silver and silver chloride nanoparticles.

The essence of the invention concerns a lactic acid bacterial strain of *Lactiplantibacillus plantarum* deposited in the Polish Collection of Microorganisms (PCM) at the Institute of Immunology and Experimental Therapy of the Polish Academy of Sciences, 12 Rudolf Weigl St. with its seat in Wroclaw, Poland, under number [B/00401] for use in the synthesis of zinc oxide, silver, silver chloride nanoparticles and hybrid systems of silver and silver chloride nanoparticles.

The essence of the invention concerns a lactic acid bacterial strain of *Limosilactobacillus fermentum* deposited in the Polish Collection of Microorganisms (PCM) at the Institute of Immunology and Experimental Therapy of the Polish Academy of Sciences, 12 Rudolf Weigl St. with its seat in Wroclaw, Poland, under number [B/00400] for use in the synthesis of zinc oxide, silver and silver chloride nanoparticles and hybrid systems of silver and silver chloride nanoparticles.

The essence of the invention also lies in the method for isolating lactic acid bacterial strains of *Latilactobacillus curvatus* or *Lactiplantibacillus plantarum* or *Limosilactobacillus fermentum* from samples of fresh cow milk characterised by the fact that a sample of fresh cow milk preferably within 2 h after milking is diluted at least once with peptone water in a ratio of 1:1 to 1:10 (preferably 10:1) and is subjected to a reduction culture on agar medium containing agar at a concentration of 10-20 g/L, ammonium citrate at a concentration of 0.5 to 5 g/L, di-Potassium hydrogen phosphate at a concentration of 0.5 to 5 g/L, glucose at a concentration of 10 to 20 g/L, magnesium sulphate at a concentration of 0, 05 to 0.5 g/L, manganese sulphate at a concentration of 0.01 to 0.1 g/L, beef extract at a concentration of 1 to 10 g/L, peptone at a concentration of 1 to 10 g/L, sodium acetate at a concentration of 0.5 to 5 g/L, yeast extract at a concentration of 0.5 to 5 g/L, with the pH of the medium in the range of 5.8-6.8, and the bacterial colonies thus obtained are transferred by means of a loop, preferably with a medium mesh to a vessel, preferably made of glass.

The essence of the invention also lies in the method for obtaining silver and silver chloride nanoparticles from a bacterial strain of lactic acid characterised in that a lactic acid bacterial strain in the form of *Latilactobacillus curvatus* deposited under [B/00399] or *Lactiplantibacillus plantarum* deposited under [B/ 00401] or *Limosilactobacillus fermentum* deposited under [B/00400] isolated according to the method described above is placed in a vessel, preferably made of glass, is inoculated with 5-10 L of liquid medium at 27-35 °C (preferably 30 °C) over 24-48 h (preferably 30 h) at centrifugation of 50-200 rpm (preferably 100 rpm) until a bacterial suspension in the range of 0.7-1 McF is obtained, it is then centrifuged at 10000-12000 rpm (preferably 12000 rpm) for 5-20 min. (preferably 15 min), filtered, silver nitrate in the form of crystals is added in the amount from 0.129gIL to 0. 645 g/L with a final concentration from 1 to 5 mM with a concentration in the range of 1-5 mM, it is stirred from 20 s to 5 min (preferably 1 min), centrifuged at 10000-12000 rpm (preferably 12000 rpm) for 5-15 min (preferably 10 min), and the solution thus obtained is incubated in the light-free medium at 27-40 °C (preferably 36 °C) for 24-36 h (preferably 30 h), it is concentrated by centrifugation in the range of 10000-12000 rpm (preferably 12000 rpm) for 15-30 min (preferably 20 min), it is centrifuged at 12000 rpm for 15-30 min (preferably 20 min) and dialysed for 3-7 days (preferably 5 days), where the medium has a composition of ammonium citrate at a concentration of 0.5-5 g/L, di-Potassium hydrogen phosphate at a concentration of 0.5 to 5 g/L, glucose at a concentration from 15 to 30 g/L, magnesium sulphate at a concentration of 0.05-0.5 g/L, manganese sulphate in a concentration of 0.01-0.1 g/L, beef extract in a concentration of 1-10 g/L, peptone in a concentration of 5-15 g/L, sodium acetate in a concentration of 0.5-5 g/L, yeast extract in a concentration of 0.5-5 g/L. Centrifugation is preferably carried out in sterile glass flasks. Filtration is preferably carried out on a 0.22µM filter preferably made of methylcellulose. Dialysis is preferably carried out on a membrane with a molecular weight limit of 3kDa.

The essence of the invention also lies in the method for obtaining hybrids of silver and silver chloride AgNPs@AgNCI nanoparticles from a bacterial strain of lactic acid characterised in that a lactic acid bacterial strain in the form of *Latilactobacillus curvatus* deposited under [B/00399] or *Lactiplantibacillus plantarum* deposited under [B/00401] or *Limosilactobacillus fermentum* deposited under [B/00400] isolated as described above is placed in a vessel, preferably made of glass, inoculated with 5-10 L of liquid medium at 27-35 °C (preferably 30 °C) over 24-48 h (preferably 30 h) at centrifugation of 50-200 rpm (preferably 100 rpm) until a bacterial suspension of 0.7-1 McF is obtained, it is then centrifuged at 10000-12000 rpm (preferably 12000) for 5-20 min (preferably 15 min), filtered, silver nitrate crystals of 0.129 g/L- 0.645 g/L with a final concentration of 1-5 mM are added, and the resulting solution incubates at 27-40 °C (preferably 36 °C) for 24-36 h (preferably 30 h), it is stirred from 20 s to 5 min, (preferably 1 min), it centrifuges at 10000-12000 rpm (preferably 12000 rpm) for 5-15 min (preferably 10 min), and the solution thus obtained is incubated in the light-free medium at 27-40 °C (preferably 36 °C) for 24-36 h (preferably 30 h) and concentrates by centrifugation at 10000-12000 rpm (preferably 12000 rpm) for 15-30 min (preferably 20 min), centrifuged at 10000-12000 rpm (preferably 12000 rpm) for 15-30 min (preferably 20 min), and is dialysed for 3-7 days (preferably 5 days), where the medium is composed of ammonium citrate at a concentration from 0.5 to 5 g/L, potassium di-phosphate at a concentration from 0.5 to 5 g/L, glucose at a concentration from 15 to 30 g/L, magnesium sulphate at a concentration from 0.05 to 0.5 g/L, manganese sulphate at a concentration from 0.01 to 0.1 g/L, beef extract at a concentration from 1 to 10 g/L, peptone at a concentration from 5 to 15 g/L, sodium acetate at a concentration from 0.5 to 5 g/L, yeast extract at a concentration from 0.5 to 5 g/L. Preferably, centrifugation is carried out in sterile glass flasks. The filtration is preferably carried out on a 0.22µM filter preferably made of methylcellulose. The dialysis is preferably carried out on a membrane with a molecular weight limit of 3kDa.

The essence of the invention also lies in the method for obtaining zinc oxide nanoparticles from a bacterial strain of lactic acid characterised in that a lactic acid bacterial strain in the form of *Latilactobacillus curvatus* deposited under [B/00399] or *Lactiplantibacillus plantarum* deposited under [B/ 00401] or *Limosilactobacillus fermentum* deposited under [B/00400] isolated as described above is placed in a vessel, preferably made of glass, is inoculated with 100-500mL of liquid medium at 27-35 °C (preferably 30°C) for 24-48 h (preferably 30h) at centrifugation from 50 to 200 rpm (preferably 100 rpm) until a bacterial suspension ranging 0.7-1 McF is obtained, it is then centrifuged at 10000-12000 rpm (preferably 12000 rpm) for 5-20 min (preferably 15 min), zinc nitrate hexahydrate in the solid form at a concentration of 0.01-10 g/mL is added, and it is synthesised while stirring for 0.5-4 h (preferably 2 h) at 40-70 °C (preferably 60 °C), it is centrifuged at 10000-12000 rpm (preferably 12000 rpm) for 15-30 min (preferably 20 min) and the supernatant thus obtained is transferred to a sterile vessel, preferably made of glass, and heated at 80-120°C (preferably 100 °C) until the liquid has fully evaporated and ZnO NCs is obtained in the powder form, preferably within 6-10 h, most preferably within 8h, which is rinsed with water, zwortexed, sonicated, centrifuged at 8000-12000 rpm (preferably 10000 rpm) at 5-35°C (preferably 20 °C) for 5-20 min (preferably 10 min) and dialysed for 3-7 days (preferably 5 days), where the medium has a composition of ammonium citrate at 5 g/L, di-Potassium hydrogen phosphate at 5 g/L, glucose at 30 g/L, magnesium sulphate at 0.5 g/L, manganese sulphate at 0.1 g/L, beef extract at 10 g/L, peptone at 15 g/L, sodium acetate at 5 g/L, yeast extract at 5 g/L. The water is preferably deionised water. Preferably, the rinsing is carried out three to ten times. Preferably, the zwitterionisation is carried out from 5 to 25 min preferably 10 min. The sonification is preferably carried out in an ultrasonic bath from 5 to 30 min preferably 20 min. Rinsing and centrifugation is preferably carried out three to ten times. The dialysis is preferably carried out on a membrane with a molecular weight limit of 3kDa.

The invention has become obvious in the examples of implementation, bearing in mind that the examples of implementations do not exhaust all possibilities under the invention.

### Example I

### 1. Culture and isolation of bacteria

Isolation of probiotic strains was carried out from fresh cow milk up to 2h after milking. For this purpose, the serial dilution method was used with peptone water at a ratio from 1:1 to 1:10, 10:1 in the example of implementation, and a reduction culture on the medium. Bacterial isolations were carried out using agar medium with a composition of:
· agar at a concentration of 10-20g/L,
· ammonium citrate at a concentration of 0.5-5 g/L,
·di-Potassium hydrogen phosphate at a concentration of 0.5-5 g/L,
·glucose at a concentration of 10-20 g/L,
· magnesium sulphate at a concentration of 0.05-0.5 g/L,
· manganese sulphate at concentrations of 0.01-0.1 g/L,
·beef extract at a concentration of 1-10 g/L,
· peptone at a concentration of 1-10 g/L,
· sodium acetate at a concentration of 0.5-5 g/L,
·yeast extract at a concentration of 0.5-5 g/L,
· pH of the medium in the range of 5.8-6.8.

A medium of the following composition was used for further culture of the isolated probiotic strains.

For silver and zinc oxide nanocomposites, a liquid medium with the composition:
· ammonium citrate at a concentration of 0.5-5 g/L,
· di- Potassium hydrogen phosphate at a concentration of 0.5-5 g/L,
·glucose at a concentration of 15-30 g/L,
·magnesium sulphate at a concentration of 0.05-05 g/L,
·manganese sulphate at a concentration of 0.01-0.1 g/L,
· beef extract at a concentration of 1-10 g/L,
·peptone at a concentration of 5-15 g/L,
·sodium acetate at a concentration of 0.5-5 g/L,
·yeast extract at a concentration of 0.5-5 g/L
·pH of the medium in the range of 5.8-6.4

### 2. Identification of bacterial strains

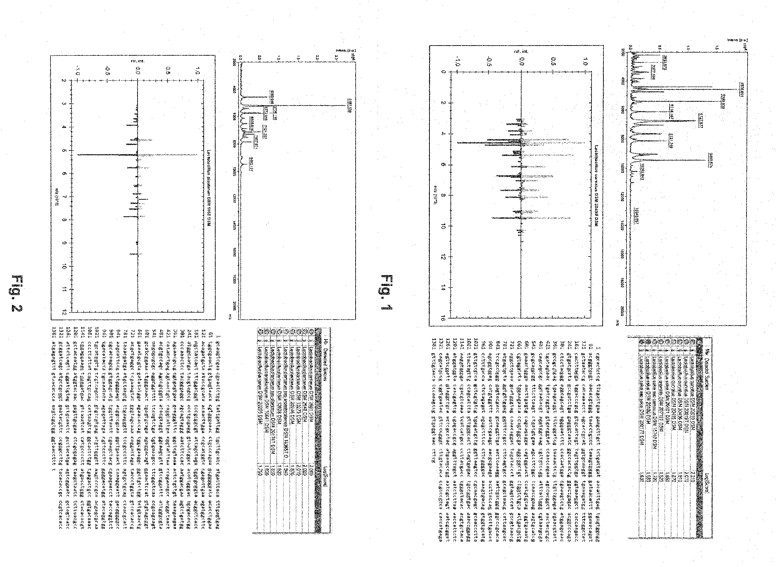

*Bacterial strains isolated from milk were identified by 16S rDNA gene sequencing and matrix-assisted laser ionisation*/*desorption spectrometric method with time-of-flight analyser (MALDI-TOF-MS) using MBT and the BIOTYPER platform (Bruker Daltonik, GmBH). The spectra for the isolated strains, including the reported match levels,* as *well* as *the 16S rDNA sequences are shown below (****Fig**.* **1-3** - **Fig. 1** MALDI-TOF-MS spectra and 16S sequences for the *Latilactobacillus curvatus* probiotic strain. **Fig. 2** MALDI-TOF-MS spectra and 16S sequences for the *Lactiplantibacillus plantarum* probiotic strain. **Fig. 3** MALDI-TOF-MS spectra and 16S sequences for the probiotic strain of *Limosilactobacillus fermentum*)*.*

The probiotic strains isolated from the milk were then deposited in the Polish Collection of Microorganisms (PCM) under the following numbers:
· *Latilactobacillus curvatus* [B/00399]
· *Limosilactobacillus fermentum* [B/00400],
· *Lactiplantibacillus plantarum* [B/00401]

### 3. Synthesis of Ag and ZnO NPs

### a. Ag NPs

Use of lactic acid bacteria strains isolated from milk strains:
· *Latilactobacillus curvatus* [B/00399]
· *Limosilactobacillus fermentum* [B/00400],
· *Lactiplantibacillus plantarum* [B/00401]

as a biologically active material of natural origin for the extracellular microbial treatment of three types of silver nanocomposites:
   · AgNPs,
   · AgNCl,
   · AgNPs H (AgNPs@AgNCl - hybride).
· Two methods of extracellular microbial synthesis were used for the above-mentioned types of silver nanocomposites:
   · direct method (synthesis of hybrid nanocomposites - AgNPs@AgNCl (AgNPs H)),
   ·modified method (synthesis and separation of AgNPs and AgNCl nanocomposites.

The culture of *Lactobacilli* strains isolated from milk is carried out at a temperature from 27 to 35°C, 30°C in the example of implementation, for 24-48 h, 30 h in the example of implementation, at a centrifugation of 100 rpm using a Biostat A bioreactor (Sartorius Stedim Biotech, Germany) to obtain a large amount of metabolites that will be naturally involved in the synthesis of nanocomposites (*stage 1*)*.* The resulting bacterial suspension ranges from 0.7 to 1 McF.

The resulting culture is centrifuged in sterile phalcones at 10000-12000 rpm, 2000 rpm in the example of implementation, for 5-20 min, 15 min in the example of implementation, to eliminate bacterial cells (*stage 2*). The resulting supernatant is filtered using a 0.22µM filter. A precursor in the form of silver nitrate crystals with a final concentration from 1 to 5 mM are added to the resulting bacterial supernatant (*stage 3*)*.*

For the *direct method,* the resulting mixture is given an additional incubation at 27- 40°C, 36°C in the example of implementation, for 24 to 36 h, 30 h in the example of implementation.

For the *modified method,* immediately after mixing the supernatant with the precursor for 1 min, the mixture is centrifuged at 10000-12000 rpm, 12000 in the example of implementation, for 5-15 min, 10 min in the example of implementation, to separate the AgNPs (supernatant) and AgNCI (precipitate) from each other.

The resulting supernatant solution and precipitate are incubated at a temperature from 27 to 40°C, 36°C in the example of implementation, for a further 24-36 h, 30 h in the example of implementation, in the light-free medium to allow full synthesis of the nanocomposites.

Once the required time was reached, all nanocomposites obtained by the direct (AgNPs@AgNCl) and modified (AgNPs and AgNCI) methods were concentrated by centrifugation at 10000-12000 rpm, 12000 in the example of implementation, for 15-30 min, 20 min in the example of implementation.

The resulting nanocomposites are cleaned with water by centrifugation at 10000-12000 rpm, 12000rpm in the example of implementation, for 5-25 min, 10 min in the example of implementation.

The pre-purified nanocomposites are dialysed for 3 to 7 days, 5 days in the example of implementation, using a membrane with a (*cut-off*) molecular weight limit of 3kDa to eliminate silver ions and low molecular weight metabolites that were not involved in the formation of the silver nanocomposites.

The silver nanocomposites thus purified are subjected to physico-chemical characterisation with a range of instrumental methods and are used for antimicrobial activity tests.

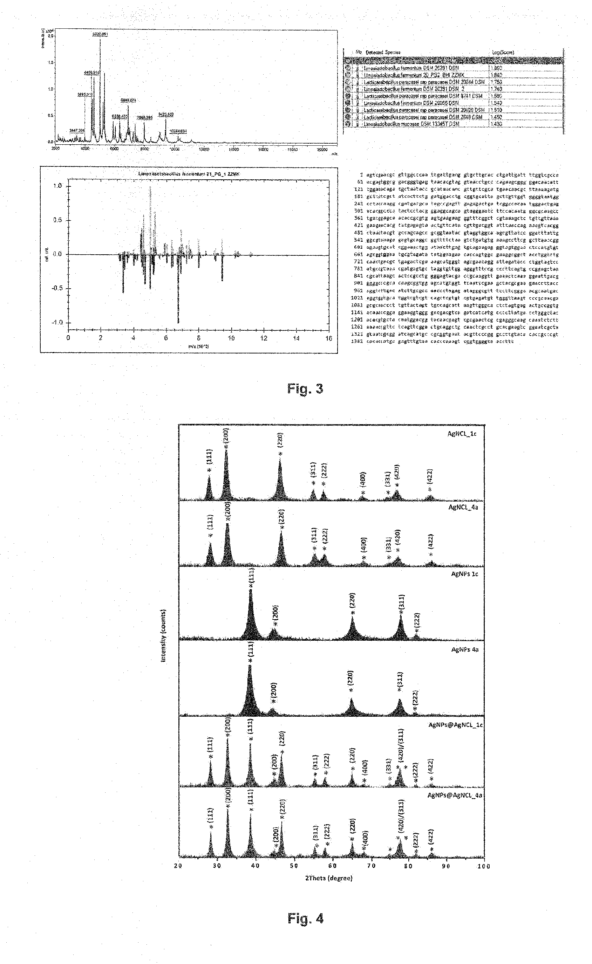

The presence of chlorides in the samples, as well as the presence of Ag-NCI and Ag-NPs H in crystalline form, was confirmed by XRD analysis (**Fig. 4** - XRD spectrum for silver nanocomposites). The diffraction signals of AgNPs, AgNCI and AgNPs H samples synthesised by both bacterial strains are similar.

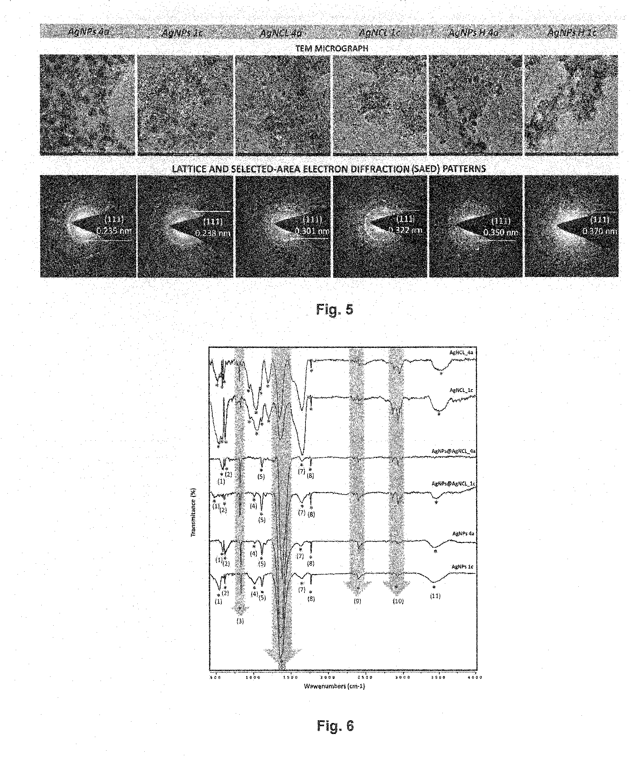

The diffraction signals of the AgNPs, AgNCI and AgNPs H samples synthesised by both bacterial strains are similar. Moreover, no additional signals were observed in the diffraction image of each sample, indicating the formation of a pure crystal structure of all nanoparticles obtained, which also proves the effective separation of AgNPs and AgNCl nanoparticles. This phenomenon was also confirmed by TEM analysis (**Fig. 5** - *Illustration of TEM micrographs, diffraction patterns (SAED) for all synthesised nanoparticles.*)*.*

Spectroscopic analysis (Fourier transform infrared spectroscopy; FT-IR) and the use of mass spectrometry (LDI-TOF-MS) showed that the silver nanocomposites are coated with an organic system (which includes, among others, amino acids (**Figs. 6** *FTIR spectra showing similarities and divergences between AgNCl and AgNPs and AgNPsH.* **and 7** *MALDI mass spectra showing MS view of discrepancies in molecular fingerprint and isotope distribution for all synthesised types of silver nanocomposites*), as well as silver clusters.

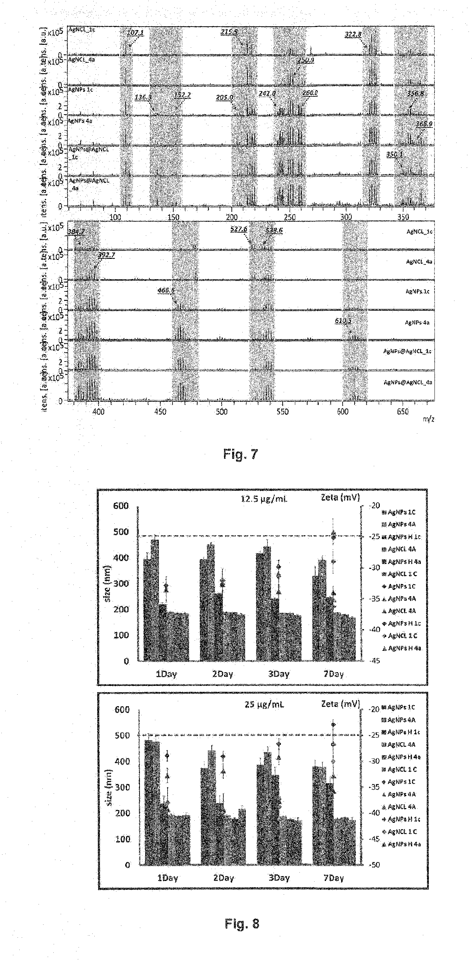

In aqueous systems, the hydrodynamic size and charge depend on the concentration of the silver nanocomposites (**Fig. 8** - *Graphs showing hydrodynamic diameter and stability of all synthesised types of silver nanocomposites over 7 days*)*.* All the nanocomposite types studied were found to have hydrodynamic diameters ranging from 176 nm to 480 nm and zeta potential values in the range from - 23 to -41 mV (**Fig. 8**).

### b. ZnO NPs

Use of lactic acid bacterial strains isolated from milk strains:
· *Latilactobacillus curvatus* [B/00399]
· *Limosilactobacillus fermentum* [B/00400],
· *Lactiplantibacillus plantarum* [B/00401]
as a biologically active material of natural origin for the extracellular microbial synthesis of zinc oxide nanoparticles (ZnO NPs).
The probiotic strains isolated from the milk are inoculated in liquid medium to obtain a bacterial suspension at acentrifugation of 50-200 rpm, 100 rpm in the example of implementation, with a density of 0.7-1 McF, 0.8 McF in the example of implementation.

The culture of the probiotic strains isolated from milk is carried out at 27-35°C, 30°C in the example of implementation, for 24-48 h, for 30 h in example of implementation.

The resulting culture is centrifuged in sterile phalcones at 10000-12000 rpm, 12000 rpm in the example of implementation, for 5-20 min, 15 min in the example of implementation, to eliminate bacterial cells.

The resulting bacterial supernatant is stored under sterile conditions until microbial synthesis of ZnO NPs begins.

Zinc nitrate hexahydrate (Zn(NO₃)₂ · 6 H₂O) is added to the resulting bacterial supernatant in a solid form and at a concentration of 0.01 - 10 g/mL.

Synthesis using the culture supernatant of probiotic strains isolated from milk and zinc nitrate hexahydrate as a precursor was carried out for 0.5-4 h, 2 h in the example of implementation at 40-70°C, 60°C in the example of implementation (*synthesis stage I*)*.*

The above synthesis step occurred with simultaneous stirring.

After stage I of the synthesis, the reaction mixture was vortexed at 10000-12000 rpm, 12000 rpm in the example of implementation for 15-30 min, 20 min in the example of implementation.

The resulting supernatant after centrifugation is then transferred to a new, sterile glass vessel.

The supernatant in a new glass vessel is heated 80-120°C, 100°C in the example of implementation, until the liquid is fully evaporated, preferably within 6-10 h, within 8 h in the example of implementation, and ZnO NCs in the powder form is obtained (*synthesis stage II*)*.*

The resulting ZnO NCs were purified by rinsing with deionised water, preferably 3 to 10 times, 5 times in the example of implementation.

The resulting ZnO NCs were zwortexed, preferably for 5-25 min, 10 min in the example of implementation, and sonicated in an ultrasonic bath, preferably for 10-30 min, 20 min in the example of implementation.

The resulting ZnO NCs were centrifuged at a velocity preferably of 8000-12000 rpm, 10000 rpm in the example of implementation, at 20°C for 10 min.

Rinsing and centrifuging operations are preferably repeated from 3 to 10 times, 5 times in the example of implementation.

Pre-purified ZnO NCs were dialysed, preferably for 3-10 days, 5 days in the example of implementation, using a membrane with a (*cut-off*) molecular weight limit of 3kDa.

The ZnO NPs thus purified were subjected to physicochemical characterisation using a range of instrumental methods, and were used for antibacterial activity tests.

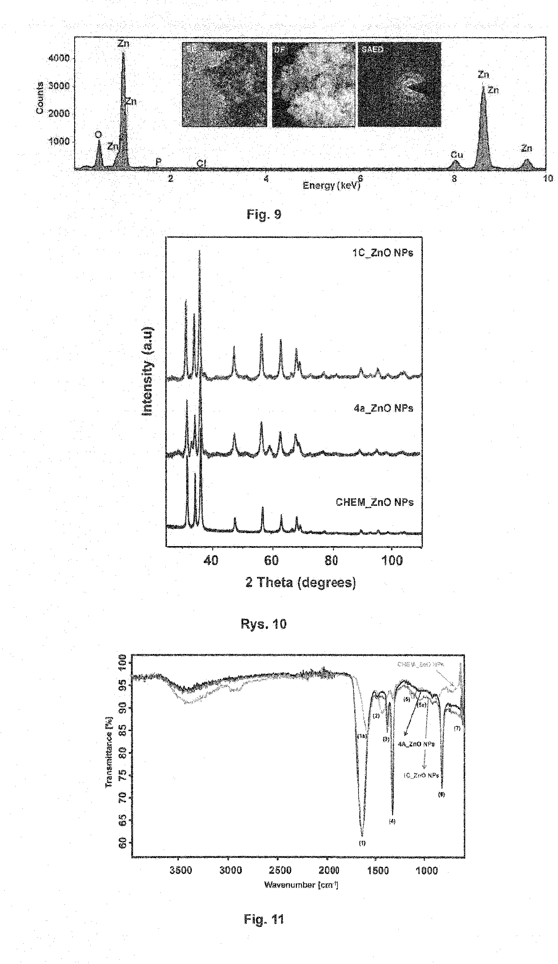

Analysis using an electron microscope (TEM) coupled to an energy dispersive X-ray detector (EDX) confirmed the presence of zinc oxide (**Fig. 9**). The diffraction image (SAED) and diffraction spectrum (XRD) shown in **Fig. 9** (Transmission electron microscopy (TEM) imaging for biologically derived ZnO NPCs; including EDX and SAED spectra) and **Fig. 10** (*Diffraction spectrum of ZnO NPs derived biologically, derived chemically (pattern*) confirmed the crystalline form of zinc oxide in the form of wurtzite with lattice constants of a = 0.3249 nm and c = 0.5208 nm (according to JCPDS card no. 36-1451, P63mc).

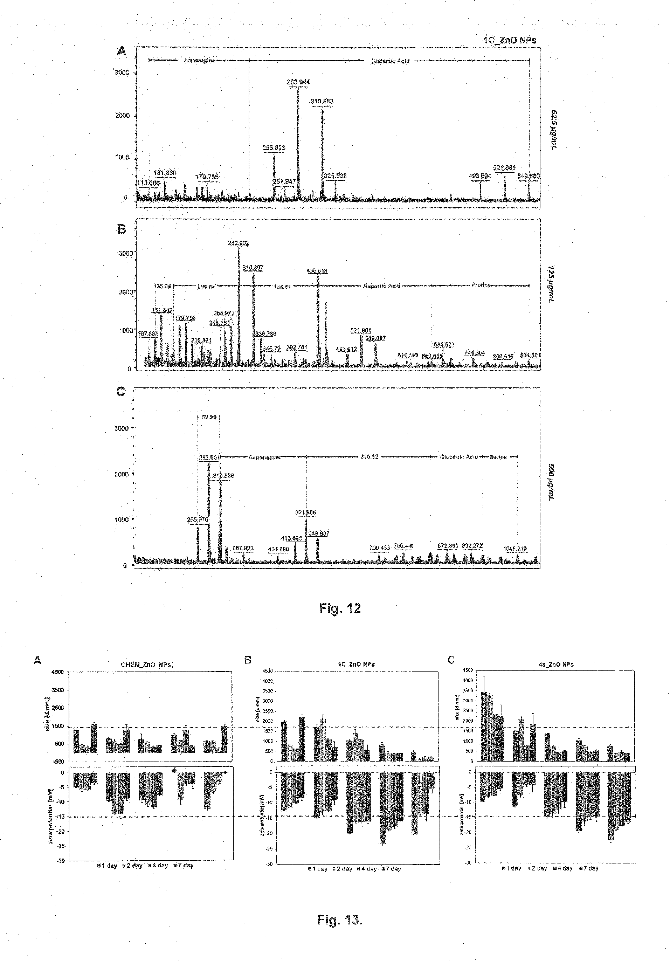

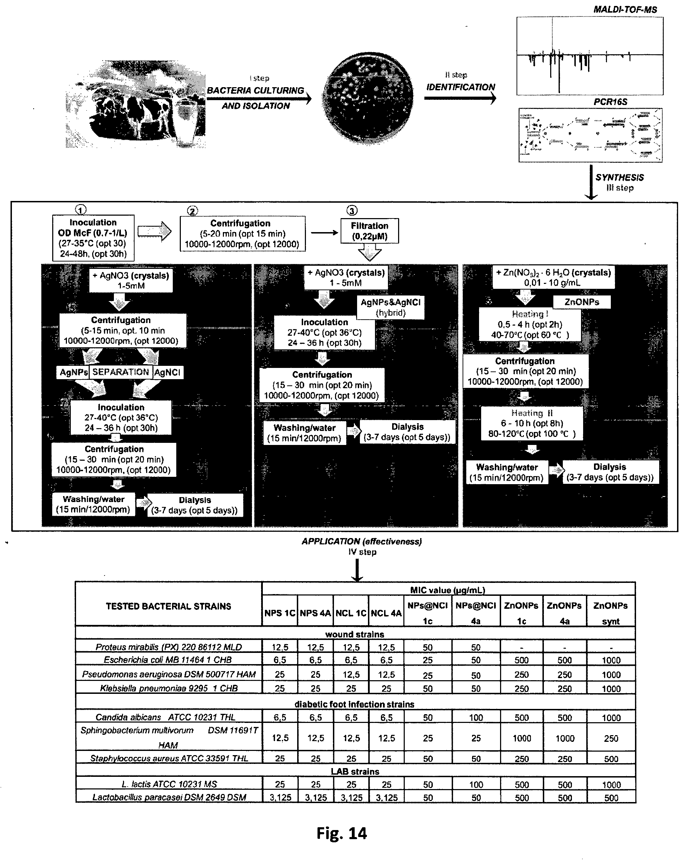

Spectroscopic analysis (Fourier transform infrared spectroscopy; FT-IR) and the use of mass spectrometry (LDI-TOF-MS) showed that the bio-ZnO NPs are coated with an organic system (which includes, among others, amino acids (**Fig. 11** - *FT-IR spectrum in the range u = 400-4000 cm-¹ for ZnO NPs derived biologically* and **Fig. 12** *Exemplary LDI-TOF-MS spectrum recorded for 1C_ZnO NPs at three concentrations*)*.*

In aqueous systems, the hydrodynamic size and charge depend on the concentration of ZnO NPs; **Fig. 13** - *Stability testing for chemical nanoparticles (A; as control), 1C_ZnO nanoparticles (B) and 4a_ZnO nanoparticles (C) is shown for hydrodynamic size (top) and zeta potential (bottom) for 7 days*)*.* The stability of ZnO NPs was confirmed by zeta potential measurements. Compared to chemically derived ZnO NPs, microbially synthesised nanoparticles show higher stability - both over time and as a function of ZnO NPs concentration. In both cases, the highest stability was determined for nanoparticles with a concentration of 62.5 µg/mL on day 1 of measurements. The ZP values at that time were -12.55± 0.49 mV for chemical ZnO NPs and -22.45±0.77 mV for ZnO NPs obtained biologically. With the passage of time (from day 1 to day 7 of measurements), the stability of bio-ZnO NPs decreases slightly - for a concentration of 125 µg/mL, the ZP value changes from -19.46±0.38 mV (day 1) to -14.95±1.34 mV (**Fig**. **14** - *Stage flow chart: bacterial culture and isolation, identification, synthesis of nano-systems and application*)*.*

### Application of Ag and ZnO NPs as an antibacterial agent

Testing of the antimicrobial activity of the resulting nanomaterials was performed for 9 microorganisms, 4 of which were isolated from hard-to-heal wounds (e.g. decubitus ulcers), 3 isolates were strains isolated from diabetic foot infections and 2 were probiotic strains. In total, 8 bacterial strains and 1 fungal strain were tested. The minimum inhibitory concentration was found to be in the range of 3.125-100 µg/ml for silver nanoparticles, while for zinc oxide nanoparticles the value was in the range of 250-1000 pg/ml. No differences were observed between AgNPs and AgNCl, while strong differences were noted between AgNPs@AgNCI (hybrid system) and separated silver nanoparticles. Furthermore, differences were noted between the nanoparticles synthesised by different strains; the AgNPs@AgNCI 4a hybrid system showed a higher MIC (minimum inhibitory concentration) than AgNPs@AgNCI 1c for Escherichia coli MB 11464 1 CHB, Pseudomonas aeruginosa DSM 500717 HAM, Candida albicans ATCC 10231 THL, L. lactis ATCC 10231 MS. In contrast, both AgNC1 1c and 4a show a slightly lower MIC value against Pseudomonas aeruginosa DSM 500717 HAM compared to both AgNPs 1c and 4a. In the case of zinc nanoparticles, strong differences were observed between the biologically and chemically synthesised ones; the chemically synthesised ones show a higher MIC value (500 and even 1000 µg/mL) with the exception of the effect against the *Sphingobacterium multivorum* DSM 11691T HAM strain, where the opposite effect was observed (a decrease in MIC value from 1000 to 250 µg/mL). The antimicrobial results for the above-described nanocarriers are summarised below.

### Example II

New lactic acid bacterial strain of *Lactobacillus curvatus* deposited in the Polish Collection of Microorganisms (PCM) at the Institute of Immunology and Experimental Therapy of the Polish Academy of Sciences, 12 Rudolf Weigl St. with its seat in Wroclaw, Poland, under number [B/00399] for use in the synthesis of zinc oxide, silver, silver chloride nanoparticles and hybrid systems of silver and silver chloride nanoparticles.

### Example III

New lactic acid bacterial strain of *Lactiplantibacillus plantarum,* deposited in the Polish Collection of Microorganisms (PCM) at the Institute of Immunology and Experimental Therapy of the Polish Academy of Sciences, 12 Rudolf Weigl St. with its seat in Wroclaw, Poland, under number [B/00401] for use in the synthesis of zinc oxide, silver, silver chloride nanoparticles and hybrid systems of silver and silver chloride nanoparticles.

### Example IV

New lactic acid bacterial strain of *Limosilactobacillus fermentum* deposited in the Polish Collection of Microorganisms (PCM) at the Institute of Immunology and Experimental Therapy of the Polish Academy of Sciences, 12 Rudolf Weigl St. with its seat in Wroclaw, Poland, under number [B/00400] for use in the synthesis of zinc oxide nanoparticles, silver, silver chloride and hybrid systems of silver nanoparticles and silver chloride.

### Example V

The method for isolating lactic acid bacterial strains of *Latilactobacillus curvatus* from samples of fresh cow milk involves diluting a sample of fresh cow milk within 1h of milking three times with peptone water at a ratio of 10:1 and subjected to a reduction culture on agar medium containing agar at 15 g/L, ammonium citrate at 2.5 g/L, di-Potassium hydrogen phosphate at 2.5 g/L, glucose at 15 g/L, magnesium sulphate at 0.25 g/L, manganese sulphate at 0.05 g/L, beef extract at a concentration of 5 g/L, peptone at a concentration of 5 g/L, sodium acetate at a concentration of 2.5 g/L, yeast extract at a concentration of 2.5 g/L, where the pH of the medium is 6 and the bacterial colonies thus obtained are transferred by means of a loop into a vessel.

The method resulted in the isolation of *Latilactobacillus curvatus.*

### Example VI

The method for isolating lactic acid bacterial strains of *Lactobacillus plantarum* from samples of fresh cow milk involves diluting a sample of fresh cow milk 15min after milking six times with peptone water at a ratio of 1:1 and subjected to a reduction culture on agar medium containing agar at 10 g/L, ammonium citrate at 0.5 g/L, di-Potassium hydrogen phosphate at 0.5 g/L, glucose at 10 g/L, magnesium sulphate at 0, 05 g/L, manganese sulphate at a concentration of 0.01 g/L, beef extract at a concentration of 1 g/L, peptone at a concentration of 1 g/L, sodium acetate at a concentration of 0.5 g/L, yeast extract at a concentration of 0.5 g/L, where the pH of the medium is 5.8 and the bacterial colonies thus obtained are transferred by means of a loop with a medium mesh into a vessel, preferably made of glass.

The method resulted in the isolation of *Lactiplantibacillus plantarum.*

### Example VII

The method for isolating lactic acid bacterial strains of *Limosilactobacillus fermentum* from samples of fresh cow milk involves diluting a sample of fresh cow milk 110 min after milking ten times with peptone water at a ratio of 1:10 and is subjected to a reduction culture on agar medium containing agar at 20 g/L, ammonium citrate at 5 g/L, di-Potassium hydrogen phosphate at 5 g/L, glucose at 20 g/L, magnesium sulphate at 0.5 g/L, manganese sulphate at a concentration of 0.1 g/L, beef extract at a concentration of 10 g/L, peptone at a concentration of 10 g/L, sodium acetate at a concentration of 5 g/L, yeast extract at a concentration of 5 g/L, where the medium pH is 6.8 and the bacterial colonies thus obtained are transferred by means of a loop with a medium mesh into a glass vessel.

The method resulted in the isolation of *Limosilactobacillus fermentum.*

It should be noted that any of the three bacterial strains, i.e. *Latilactobacillus curvatus, Lactiplantibacillus plantarum* and *Limosilactobacillus fermentum* can be used in examples V, VI and VII.

### Example VIII

The method for obtaining silver and silver chloride nanoparticles from a lactic acid bacterial strain involves placing a lactic acid bacterial strain in the form of *Latilactobacillus curvatus* deposited under [B/ 00399] isolated according to Example V in a vessel and inoculating a liquid medium of 7L at 30 °C for 30 h at centrifugation of 100 rpm until a bacterial suspension of 0.85 McF is obtained, it is then centrifuged at 12000 rpm for 15 min, filtered, silver nitrate crystals of 0.35 g/L with a final concentration of 3 mM are added, it is stirred for 2 min, centrifuged at 12000 rpm for 10 min, and the solution thus obtained is incubated in the light-free medium at 36 °C for 30 h, it is concentrated by centrifugation at 12000 rpm for 20 min, centrifuged at 12000 rpm for 20 min and dialysed for 5 days, where the medium is composed of ammonium citrate at 2 g/L, di-Potassium hydrogen phosphate at 2 g/L, glucose at 22 g/L, magnesium sulphate at 0.2 g/L, manganese sulphate at 0.5 g/L, beef extract at 4 g/L, peptone at 11 g/L, sodium acetate at 3 g/L, yeast extract at 2 g/L.

Silver and silver chloride nanoparticles with antibacterial properties against Gram-positive and Gram-negative bacteria were obtained by this method.

### Example IX

The method for obtaining silver and silver chloride nanoparticles from a lactic acid bacterial strain involves placing a lactic acid bacterial strain in the form of *Lactiplantibacillus plantarum* deposited under [B/00401] isolated according to Example VI in a glass vessel and inoculating a liquid medium of 5L at 27 °C for 24 h at centrifugation of 50 rpm until a bacterial suspension of 0.7 McF is obtained, it is then centrifuged at 10000 rpm for 5 min, filtered, silver nitrate crystals of 0.129g/L with a final concentration of 1 mM are added, it is mixed for 20 s, centrifuged at 10000 rpm for 5 min, and the solution thus obtained is incubated in light-free medium at 27° C for 24h, it is concentrated by centrifugation at 10000 rpm for 15 min, centrifuged at 10000 rpm for 15 and dialysed for 3 days, where the medium is composed of ammonium citrate at 0.5 g/L, di-Potassium hydrogen phosphate at 0.5 g/L, glucose at 15 g/L, magnesium sulphate at 0.05 g/L, manganese sulphate at 0.01 g/L, bovine extract at 1 g/L, peptone at 5 g/L, sodium acetate at 0.5 g/L, yeast extract at 0.5 g/L.

Silver and silver chloride nanoparticles with antibacterial properties against Gram-positive and Gram-negative bacteria were obtained by this method.

### Example X

The method for obtaining silver and silver chloride nanoparticles from a lactic acid bacterial strain involves placing a lactic acid bacterial strain in the form of *Limosilactobacillus fermentum* deposited under [B/00400] isolated according to Example VII in a glass vessel and inoculating a liquid medium of 10L at 35 °C for 48 h at centrifugation of 200 rpm until a bacterial suspension of 1 McF is obtained, it is then centrifuged at 12,000 rpm for 20 min, filtered, silver nitrate crystals of 0.645 g/L with a final concentration of 5 mM are added, it is mixed for 5 min, centrifuged at 12,000 rpm for 15 min, and the solution is incubated in a light-free medium at 40°C for 36 h, concentrated by centrifugation at 12,000 rpm for 30 min, centrifuged at 8,000 rpm for 30 min and dialysed for 7 days, where the medium has a composition of ammonium citrate at a concentration of 0.5 to 5 g/L, potassium di-Potassium hydrogen phosphate at a concentration of 0.5 to 5 g/L, glucose at a concentration of 15 to 30 g/L, magnesium sulphate at a concentration of 0.05 to 0.5 g/L, manganese sulphate at a concentration of 0.01 to 0.1 g/L, beef extract at a concentration of 1 to 10 g/L, peptone at a concentration of 5 to 15 g/L, sodium acetate at a concentration of 0.5 to 5 g/L, yeast extract at a concentration of 0.5 to 5 g/L.

Silver nanoparticles and silver chloride nanoparticles with antibacterial properties against Gram-positive and Gram-negative bacteria were obtained by this method.

### Example XI

Example XI differs from X in that centrifugation is carried out in sterile glass flasks, filtration is carried out on a 0.22µM methylcellulose filter and dialysis is carried out on a membrane with a molar mass limit of 3kDa.

Any of three bacterial strains can be used, i.e. *Latilactobacillus curvatus, Lactiplantibacillus plantarum* and *Limosilactobacillus fermentum* in examples VIII, IX and X,

### Example XII

The method for obtaining hybrids of silver and silver chloride AgNPs@AgNCI nanoparticles from a lactic acid bacterial strain involves placing a lactic acid bacterial strain in the form of *Latilactobacillus curvatus* deposited under [B/00399] isolated according to Example V in a dish and inoculating a liquid medium of 8L liquid medium at 30 °C for 30h at centrifugation of 100 rpm until a bacterial suspension of 0.9 McF is obtained, it is then centrifuged at 12000 rpm for 15 min, filtered, silver nitrate crystals of 0.454 g/L with a final concentration of 3 mM are added, it is incubated at 36 °C for 30 h, stirred for 1 min, centrifuged at 12000 rpm for 10 min, and the solution thus obtained is incubated in light-free medium at 36 °C for 30 h, concentrated by centrifugation at 12000 rpm for 20 min, centrifuged at 12000 rpm for 20 min and dialysed for 5 days, where the medium is composed of ammonium citrate at 2 g/L, di-Potassium hydrogen phosphate at 2 g/L, glucose at 24 g/L, magnesium sulphate at 0.3 g/L, manganese sulphate at 0.03 g/L, beef extract at 7 g/L, peptone at 9 g/L, sodium acetate at 3.1 g/L, yeast extract at 3.4 g/L.

A hybrid of silver and silver chloride AgNPs@AgNCI nanoparticles with antibacterial properties against Gram-positive and Gram-negative bacteria was obtained by this method.

### Example XIII

The method for obtaining silver and silver chloride AgNPs@AgNCl nanoparticle hybrids from a lactic acid bacterial strain involves placing a lactic acid bacterial strain in the form of *Lactiplantibacillus plantarum* deposited under [B/00401] isolated according to Example VI in a glass vessel and inoculating a liquid medium of 5L at 27 °C for 24 h at 50 rpm centrifugation until a bacterial suspension of 0. 7 McF, it is centrifuged at 10000 rpm for 5 h, filtered, silver nitrate crystals of 0. 129g/L with a final concentration of 1 mM are added, it is incubated at 27°C for 24 h, stirred for 20 s, centrifuged at 10000 rpm for 5 h, and the solution thus obtained is incubated in light-free medium at 27° C for 24 h, concentrated by centrifugation at 10000 rpm for 15 min, centrifuged at 10000 rpm for 15 min and dialysed for 3 days, where the medium has a composition of ammonium citrate at 0.5 g/L, potassium di-Potassium hydrogen phosphate at 0.5 g/ L, glucose at 15 g/L, magnesium sulphate at 0.05 g/L, manganese sulphate at 0.01 g/L, beef extract at 1 g/L, peptone at 5 g/L, sodium acetate at 0.5 g/L, yeast extract at 0.5 g/L.

A hybrid of silver and silver chloride AgNPs@AgNCI nanoparticles with antibacterial properties against Gram-positive and Gram-negative bacteria was obtained by this method.

### Example XIV

The method for obtaining hybrids of silver and silver chloride AgNPs@AgNCI nanoparticles from a lactic acid bacterial strain involves placing a lactic acid bacterial strain in the form of *Limosilactobacillus fermentum* deposited under [B/00400] isolated according to Example VII in a glass vessel and inoculating a liquid medium of 10 at 35°C for 48 h at 200 rpm centrifugation until a bacterial suspension of 1 McF is obtained, it is centrifuged at 12000 rpm for 20 min, filtered, silver nitrate crystals at 0. 645 g/L with a final concentration of 5 mM are added, it is incubated at 40°C for 36 h, stirred for 5 min, centrifuged at 12000 rpm for 15 min, and the solution thus obtained is incubated in light-free medium at 40°C for 36 h, concentrated by centrifugation at 12000 rpm for 30 min, centrifuged at 6000 rpm for 30 min and dialysed for 7 days, wher the medium has a composition of ammonium citrate at 5 g/L, di-Potassium hydrogen phosphate at 5 g/L, glucose at 30 g/L, magnesium sulphate at 0.5 g/L, manganese sulphate at 0.1 g/L, bovine extract at 10 g/L, peptone at 15 g/L, sodium acetate at 5 g/L, yeast extract at 5 g/L.

A hybrid of silver and silver chloride AgNPs@AgNCI nanoparticles with antibacterial properties against Gram-positive and Gram-negative bacteria was obtained by this method.

### Example XV

Example XV differs from Example XIV in that centrifugation is carried out in sterile glass flasks, filtration is carried out on a 0.22µM methylcellulose filter and dialysis is carried out on a membrane with a molar mass limit of 3kDa.

In Examples VIII, IX, X, XII, XII and XIV, any of three bacterial strains, i.e. *Latilactobacillus curvatus, Lactiplantibacillus plantarum* and *Limosilactobacillus fermentum,* can be used.

### ExampleXVI

The method for obtaining zinc oxide nanoparticles from a lactic acid bacterial strain involves placing a lactic acid bacterial strain in the form of *Lactobacillus curvatus* deposited under [B/00399] isolated according to Example V in a vessel and inoculating a liquid medium of 300 mL at 30°C for 30 h at 100 rpm centrifugation until a bacterial suspension of 0.85 McF is obtained, it is then centrifuges at 12000 rpm for 15 min, zinc nitrate hexahydrate solid at 6 g/mL is added and synthesised while stirring for 2 h at 60°C, it is centrifuged at 12000 rpm for 20 min and the supernatant thus obtained is transferred to a sterile vessel and heated at 100°C until the liquid is fully evaporated and ZnO NCs is obtained in the powder form within 8 h, which powder is rinsed with water, zwortexed under sonication, centrifuged at 10000 rpm at 20°C for 10 min and dialysed for 5 days, where the medium is composed of: ammonium citrate at 5 gl L, di-Potassium hydrogen phosphate at 5 g/L, glucose at 30 g/L, magnesium sulphate at 0.5 g/L, manganese sulphate at 0.1 g/L, beef extract at 10 g/L, peptone at 15 g/L, sodium acetate at 5 g/L, yeast extract at 5 g/L.

Zinc oxide nanoparticles with antibacterial properties against Gram-positive and Gram-negative

### Example XVII

The method for obtaining zinc oxide nanoparticles from a lactic acid bacterial strain involves placing a lactic acid bacterial strain in the form of *Lactiplantibacillus plantarum* deposited under [B/00401] isolated according to Example VI in a glass vessel and inoculating a liquid medium of 100 mL at 27 °C for 24 h at 50 rpm centrifugation until a bacterial suspension of 0.7 McF is obtained, it is then centrifuged at 10000 rpm for 5 min, zinc nitrate hexahydrate solid at 0.01 g/mL is added and synthesised while stirring for 0.5 at 40 °C, it is centrifuged at 10000 rpm for 15 min and the supernatant thus obtained is transferred to a sterile glass vessel and heated at 80 °C until the liquid is fully evaporated and ZnO NCs is obtained in the powder form within 6h, which powder is rinsed with water, zwortexed, sonicated, centrifuged at 8000 rpm at 5°C for 5 min and dialysed within 3 h, where the medium is composed of: ammonium citrate at 5 g/L, di-Potassium hydrogen phosphate at 5 g/L, glucose at 30 g/L, magnesium sulphate at 0.5 g/L, manganese sulphate at 0.1 g/L, beef extract at 10 g/L, peptone at 15 g/L, sodium acetate at 5 g/L, yeast extract at 5 g/L.

Zinc oxide nanoparticles with antibacterial properties against Gram-positive and Gram-negative bacteria were obtained by the method.

### Example XVIII

The method for obtaining zinc oxide nanoparticles from a lactic acid bacterial strain involves placing a lactic acid bacterial strain in the form of *Limosilactobacillus fermentum* deposited under [B/00400] isolated according to Example VII in a glass vessel and inoculating a liquid medium of 500mL at 35°C for 48 h at 200 rpm centrifugation until a bacterial suspension of 1 McF is obtained, it is then centrifuged at 12000 rpm for 20 min, zinc nitrate hexahydrate solid at 10 g/mL is added and synthesised while stirring for 4 h at 70°C, it is centrifuged at 12000 rpm for 30 min and the supernatant thus obtained is transferred to a sterile glass vessel and heated at 120°C until the liquid is fully evaporated and ZnO NCs is obtained in the powder form within 10 h, which powder is rinsed with water, zwortexed, sonicated, centrifuged at 12000 rpm at 35°C for 20 min and dialysed for 7 days, where the medium is composed of: ammonium citrate at 5 g/L, di-Potassium hydrogen phosphate at 5 g/L, glucose at 30 g/L, magnesium sulphate at 0.5 g/L, manganese sulphate at 0.1 g/L, beef extract at 10 g/L, peptone at 15 g/L, sodium acetate at 5 g/L, yeast extract at 5 g/L.

Zinc oxide nanoparticles with antibacterial properties against Gram-positive and Gram-negative bacteria were obtained by this method.

### Example XIX

Example XIX differs from Example XVIII in that the water is deionised water, the rinsing is carried out from three to ten times, the zwortexing is carried out from 5 to 25 min preferably 10 min, sonification is carried out in an ultrasonic bath preferably from 5 to 30 min preferably 20 min, the rinsing and centrifugation is carried out from three to ten times preferably eight times and that the dialysis is carried out on a membrane with a molecular weight limit of 3kDa

Any of the three bacterial strains, i.e. *Latilactobacillus curvatus, Lactiplantibacillus plantarum* and *Limosilactobacillus fermentum,* can be used in examples VIII, IX, X, XVI, XVII and XVIII.

## Claims

1. Lactic acid bacterial strain *Latilactobacillus curvatus* deposited in the Polish Collection of Microorganisms (PCM) in the Institute of Immunology and Experimental Therapy of the Polish Academy of Sciences, 12 Rudolf Weigl Street with its seat in Wroclaw, Poland, under number [B/00399] for use in the synthesis of zinc oxide, silver, silver chloride nanoparticles and hybrid systems of silver and silver chloride nanoparticles.

2. Lactic acid bacterial strain *Lactiplantibacillus plantarum* deposited in the Polish Collection of Microorganisms (PCM) in the Institute of Immunology and Experimental Therapy of the Polish Academy of Sciences, 12 Rudolf Weigl Street with its seat in Wroclaw, Poland, under number [B/00401] for use in the synthesis of zinc oxide, silver, silver chloride nanoparticles and hybrid systems of silver and silver chloride nanoparticles.

3. Lactic acid bacterial strain *Limosilactobacillus fermentum* deposited in the Polish Collection of Microorganisms (PCM) in the Institute of Immunology and Experimental Therapy of the Polish Academy of Sciences, 12 Rudolf Weigl Street with its seat in Wroclaw, Poland, under number [B/00400] for use in the synthesis of zinc oxide nanoparticles, silver, silver chloride nanoparticles and hybrid systems of silver and silver chloride nanoparticles.

4. The method for isolating lactic acid bacterial strains *Latilactobacillus curvatus* or *Lactiplantibacillus plantarum* or *Limosilactobacillus fermentum* from samples of fresh cow milk is **characterised by** diluting a sample of fresh cow milk preferably within 2 h of milking at least once with peptone water in a ratio of 1:1 to 1:10 preferably 10:1 and is subjected to a reduction culture on agar medium containing agar at a concentration of 10-20g/L, ammonium citrate at a concentration of 0.5 to 5 g/L, di-Potassium hydrogen phosphate at a concentration of 0.5 to 5 g/L, glucose at a concentration of 10 to 20 g/L, magnesium sulphate at a concentration of 0.05 to 0.5 g/L, manganese sulphate at a concentration of 0.01 to 0.1 g/L, beef extract at a concentration of 1 to 10 g/L, peptone at a concentration of 1 to 10 g/L, sodium acetate at a concentration of 0.5 to 5 g/L, yeast extract at a concentration of 0.5 to 5 g/L, with the pH of the medium ranging from 5. 8 to 6.8 and the bacterial colonies thus obtained are transferred by means of a loop, preferably with a medium mesh to a vessel, preferably made of glass.

5. The method for obtaining silver and silver chloride nanoparticles from lactic acid bacterial strain **characterised by** the following: lactic acid bacterial strain in the form of *Latilactobacillus curvatus* deposited under [B/00399] or *Lactiplantibacillus plantarum* deposited under [B/00401] or *Limosilactobacillus fermentum* deposited under [B/00400], isolated in accordance with claim 4, is placed in a vessel (preferably made of glass), is inoculated with 5-10 L of liquid medium at 27-35 °C (preferably 30 °C) for 24-48 h (preferably 30 h) at centrifugation ranging 50- 200 rpm (preferably 100 rpm) until a bacterial suspension ranging 0.7-1 McF is obtained, it is then centrifuged at 10000-12000 rpm (preferably 12000) for 5-20 min ( preferably 15 min), filtered, silver nitrate crystals from 0.129g/L to 0. 645 g/L with a final concentration of 1-5 mM are added, it is stirred from 20 s to 5 min (preferably 1 min), centrifuged at 10000-12000 rpm (preferably 12000 rpm) from 5-15 min (preferably 10 min), and the solution thus obtained is incubated in a light-free medium at 27-40 °C (preferably 36°C) for 24-36 h (preferably 30 h), it is concentrated by centrifugation at 10000-12000 rpm (preferably 12000) for 15-30 min (preferably 20 min), centrifuged at 12000 rpm (preferably 15-30 min) for 15-30 min (preferably 20 min) and dialysed for 3-7 days (preferably 5 days), where the medium has a composition of ammonium citrate at a concentration of 0.5 to 5 g/L, di-Potassium hydrogen phosphate at a concentration of 0.5 to 5 g/L, glucose at a concentration of 15 to 30 g/L, magnesium sulphate at a concentration of 0.05 to 0.5 g/L, manganese sulphate at a concentration of 0.01 to 0.1 g/L, beef extract at a concentration of 1 to 10 g/L, peptone at a concentration of 5 to 15 g/L, sodium acetate at a concentration of 0.5 to 5 g/L, yeast extract at a concentration of 0.5 to 5 g/L.

6. Method under claim 5 is **characterised by** conducting centrifugation in sterile glass flasks.

7. The method under claims 5 or 6 is **characterised by** conducting filtration on a 0.22µM filter, preferably made of methylcellulose.

8. The method under claims 5, 6 or 7 is **characterised by** conducting dialysis on a membrane with a molecular weight limit of 3kDa.

9. The method for obtaining hybrids of silver and silver chloride AgNPs@AgNCI nanoparticles from a lactic acid bacterial strain, **characterised by** the following: a lactic acid bacterial strain in the form of *Latilactobacillus curvatus* deposited under [B/00399] or *Lactiplantibacillus plantarum* deposited under [B/ 00401] or *Limosilactobacillus fermentum* deposited under [B/00400] isolated in accordance with claim 4 is placed in a vessel (preferably made of glass), is inoculated with 5-10 L of liquid medium at 27-35 °C (preferably 30 °C) for 24-48 h (preferably 30 h) at centrifugation ranging 50-200 rpm (preferably 100 rpm) until a bacterial suspension ranging 0. 7 to 1 McF is obtained, it is then centrifuged at 10000-12000 rpm (preferably 12000) for 5-20 min (preferably 15 min), filtered, silver nitrate crystals of 0.129g/L to 0. 645 g/L with a final concentration ranging 1-5 mM are added, it is incubated at 27- 40 °C (preferably 36°C) for 24-36 h (preferably 30 h), stirred from 20 s to 5 min preferably (1 min), centrifuged at 10000-12000 rpm (preferably 12000) for 5-15 min (preferably 10 min), it is then centrifuged at 10000-12000 rpm (preferably 12000) for 5-20 min (preferably 15 min), filtered, silver nitrate crystals of 0.129g/L to 0. 645 g/L with a final concentration ranging 1-5 mM are added, it is incubated at 27- 40 °C (preferably 36°C) for 24-36 h (preferably 30 h), stirred from 20 s to 5 min preferably (1 min), centrifuged at 10000-12000 rpm (preferably 12000) for 5-15 min (preferably 10 min), and thus obtained solution is incubated on an incubation medium in a light-free medium at 27-40°C (preferably 36°C) for 24-36 h (preferably 30 h), it is concentrated by centrifugation at 10000-12000 rpm (preferably 12000) for 15-30 min (preferably 20 min), centrifuged (preferably 12000 rpm) for 15-30 min (preferably 20 min) and is dialysed for 3-7 days (preferably 5 days), where the medium has a composition of ammonium citrate at a concentration of 0.5 to 5 g/L, di-Potassium hydrogen phosphate at a concentration of 0.5 to 5 g/L, glucose at a concentration of 15 to 30 g/L, magnesium sulphate at a concentration of 0.05 to 0.5 g/L, manganese sulphate at a concentration of 0.01 to 0.1 g/L, beef extract at a concentration of 1 to 10 g/L, peptone at a concentration of 5 to 15 g/L, sodium acetate at a concentration of 0.5 to 5 g/L, yeast extract at a concentration of 0.5 to 5 g/L.

10. The method under claim 9 is **characterised by** conducting centrifugation in sterile glass flasks.

11. The method under claim 9 or claim 10 is **characterised by** conducting filtration on a 0.22µM filter, preferably made of methylcellulose.

12. The method under claim 9, claim 10 or claim 11 is **characterised by** conducting dialysis on a membrane with a molecular weight limit of 3kDa.

13. The method for obtaining zinc oxide nanoparticles from a lactic acid bacterial strain **characterised by** the following: a lactic acid bacterial strain in the form of *Latilactobacillus curvatus* deposited under [B/ 00399] or *Lactiplantibacillus plantarum* deposited under [B/00401] or *Limosilactobacillus fermentum* deposited under [B/00400] isolated in accordance with claim 4 is placed in a vessel (preferably made of glass), is inoculated with 100-500 mL of liquid medium at 27-35 °C (preferably 30 °C) for 24-48 h (preferably 30 h) at centrifugation ranging 50-200 rpm (preferably 100 rpm) until a bacterial suspension ranging 0.7-1 McF is obtained, it is centrifuged at 10000-12000 rpm (preferably 12000 rpm) for 5-20 min (preferably 15 min), zinc nitrate hexahydrate in the solid form at concentrations ranging 0.01-10 g/mL is added and simultaneously stirred for 0.5-4 h (preferably 2 h) at 40-70°C (preferably 60°C), it is centrifuged at 10000-12000 rpm (preferably 12000 rpm) for 15-30 min (preferably 20 min) and the supernatant is transferred to a sterile vessel (preferably made of glass) and heated at 80-120°C (preferably 100 °C) until the liquid has fully evaporated and it is synthesised by simultaneous stirring for 0.5-4 h (preferably 2 h) at 40-70°C (preferably 60 °C), centrifuged at 10000-12000 rpm (preferably 12000 rpm) for 15-30 min (preferably 20 min) and the supernatant thus obtained is transferred to a sterile vessel (preferably made of glass) and heated at 80-120 °C (preferably 100 °C) until the liquid is fully evaporated and ZnO NCs is obtained in the powder form preferably with 6-10 h (most preferably within 8h), which powder is rinsed with water, zwortexed, sonicated, centrifuged at 8000-12000 rpm (preferably 10000 rpm) at 5-35 °C (preferably 20 °C) for 5-20 min (preferably 10 min) and dialysed for 3-7 days (peferably 5 days), where the medium has a composition of ammonium citrate at 5 g/L, di-Potassium hydrogen phosphate at 5 g/L, glucose at 30 g/L, magnesium sulfate at 0.5 g/L, manganese sulfate at 0.1 g/L, beef extract at 10 g/L, peptone at 15 g/L.

14. The method under claim 13 is **characterised in that** the water is deionized water.

15. The method under claim 13 or claim 14 is **characterised by** performing rinsing from three to ten times.

16. The method under claims 13, 14 or 15 is **characterised by** performing zwortexing from 5-25 min, preferably 10 min.

17. The method under claims 13, 14, 15 or 16 is **characterised by** performing sonification is carried out in an ultrasonic bath preferably from 5 to 30 min preferably 20 min.

18. The method under claims 13,14,15,16 or 17 is **characterised by** performing rinsing and centrifugation three to ten times.

19. The method under claims 13,14,15,16,17 or 18 is **characterised by** performing dialysis on a membrane with a molecular weight limit of 3kDa.
